# EUROPÄISCHE PATENTANMELDUNG

(11) **EP 2 005 936 A1**
(43) Veröffentlichungstag der Anmeldung: **24.12.2008**
(21) Anmeldenummer: 08005738.3
(22) Anmeldetag: 27.03.2008
(51) Int. Cl.: A61K 8/06, A61K 8/22, A61K 8/42, A61Q 19/00

(54) **Kosmetische oder dermatologische Zubereitungen mit einem Gehalt an molekularem Sauerstoff und Panthenol**

(30) Priorität: 14.06.2007 DE 102007028022
(71) Anmelder: Beiersdorf AG, 20253 Hamburg (DE)
(72) Erfinder: Bleckmann, Andreas, 22926 Ahrensburg (DE); Treu, Jens, 22844 Norderstedt (DE); Mummert, Christopher, 29553 Bienenbüttel (DE); Fey, Sven, 22397 Hamburg (DE); Rathsack, Jessica, 21614 Buxtehude (DE); Möllgaard, Svenja, Lena, 22301 Hamburg (DE)
(74) Vertreter: Wilke, Jochen

(57) **Zusammenfassung**

Zubereitungen mit einem Gehalt an molekularem Sauerstoff, ferner enthaltend Panthenol.

## Beschreibung

Die Erfindung umfasst kosmetische oder dermatologische Zubereitung mit einem Gehalt an molekularem Sauerstoff und Panthenol.

Die Stabilität der mit Sauerstoff beladenen Zubereitungen und die Sauerstoffverfügbarkeit aus den Zubereitungen ist verbessert.

Gashaltige kosmetische Zubereitungen sind an sich bekannt und bereits in zahlreichen Patenten beschrieben worden. Als Gase werden beispielsweise Sauerstoff, fluorierte Gase, Kohlendioxid, Luft, Stickstoff und dergleichen verwendet.

Eine Möglichkeit zur Stabilisierung von Gasen in kosmetischen oder dermatologischen Zubereitungen besteht z. B. darin, eine O/W-Emulsion herzustellen, welche anschließend mit Gas beaufschlagt wird. Derartige Formulierungen sind auch als Mousse bekannt (beispielsweise WO 2002-074258-A1). Nachteilig an diesen Zubereitungen des Standes der Technik ist, dass das Gas nur durch den Einsatz von Emulgatoren stabilisiert werden kann. Ferner nachteilig ist, dass das Gas bei der topischen Applikation oder bereits bei Lagerung bei 40 °C oder mehr (beispielsweise im Auto oder am Strand) in die Atmosphäre entweichen kann. Ferner ist die Beladungskapazität derartiger Zubereitungen meistens eher gering, so dass sich physiologische Effekte nach der Applikation nicht einstellen.

Aus dem Stand der Technik sind kosmetische Zubereitungen auf Basis von O/W-Emulsionen bekannt, die Sauerstoff enthalten.

Die WO 02/05754 beschreibt extern applizierbare Zubereitungen, die einen Sauerstoffträger enthalten, der in einer lipoiden Emulsion molekulardispers eingearbeitet ist, sowie deren Verwendung zur externen Behandlung / Prävention von Sauerstoffmangelzuständen der Haut.

Die WO 02/05754 offenbart O/W-Emulsionen, die als einen Sauerstofftransport durch die Lipidschicht der Haut adäquat gewährleisten sollen.

In der US 5851544 wird beschrieben, dass Hautpflegezubereitungen, welche das Hautsauerstoffniveau erhöhen, wünschenswert seien, da der Sauerstoffgehalt in der Haut niedriger sei als in anderen Körperregionen und darüber hinaus mit zunehmendem Alter abnehme. Aufgabe der US 5851544 ist es daher, den Sauerstoffgehalt der Haut langfristig zu steigern bzw. Sauerstoffmangelzustände in der Haut zu beheben.

In der DE 10333710 werden kosmetische, dermatologische oder pharmazeutische Zubereitungen auf Basis von Lipiden und/oder Lipid/Wachs-Gemischen, welche ein Gas bzw. ein Gasgemisch enthalten, beschrieben.

Die DE 10342449, DE 102005011498, DE 102005011457 und DE 102206011459 offenbaren kosmetische Zubereitungen enthaltend molekularen Sauerstoff. Auch hierin werden bevorzugt O/W-Emulsionen beschrieben. Die Herstellung der sauerstoffhaltigen O/W-Emulsionen erfolgt indem eine Vordispergierung des anorganischen Gelbildners und Quellung des Hydrokolloides unter Rühren in der Wasserphase stattfindet. Eine Vereinigung der auf 75 °C aufgeheizten Fettphase mit der auf 70 °C aufgeheizten Wasserphase und Zugabe der partikulären hydrophoben, hydrophobisierten Festkörpersubstanzen unter Rühren. Es erfolgt dann Homogenisierung bei 65 °C und 45 min Rühren unter Vakuum und Kühlung. Die Zugabe der Additive erfolgt bei 30 °C, die Homogenisierung bei 27 °C. Anschließend wird die Begasung der Emulsion bei 7-8 bar mit Sauerstoff durchgeführt.

Die Herstellung sauerstoffhaltiger kosmetischer Zubereitungen und insbesondere der Schritt der Sauerstoffzufuhr erfolgt in allen aufgeführten Druckschriften des Standes der Technik dabei unter Kühlung, bei Raumtemperatur bzw. bei maximal 30°C.

Nachteilig an den bekannten Zubereitungen ist, dass nach Einarbeitung des molekularen Sauerstoffs, die Zubereitungen nicht stabil lagerfähig sind, eine geringe Sauerstoffbeladungskapazität aufweisen und lediglich geringe Sauerstoffverfügbarkeit aufweisen.

Wünschenswert ist es daher eine kosmetische Zubereitung bereit zu stellen, die molekularen Sauerstoff enthält und die eine für Kosmetika ausreichende Lagerfähigkeit aufweist. Ebenso ist es wünschenswert ein Verfahren zur Herstellung sauerstoffhaltiger Emulsionen zur Verfügung zu haben, mit dem eine kosmetisch anwendbare und lagerfähige Zubereitung herstellbar ist.

Unter kosmetischer Hautpflege ist in erster Linie zu verstehen, daß die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Wünschenswert ist es auch Produkte zur Pflege der auf natürliche Weise gealterten Haut, sowie zur Behandlung der Folgeschäden der Lichtalterung zur Verfügung zu stellen.

Produkte zur Pflege gealterter Haut sind an sich bekannt. Sie enthalten z. B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Produkte zum Schutz vor schädlichen Oxidationsprozessen in der Haut sind an sich bekannt. Auch Produkte, welche die Sauerstoff-Versorgung der Haut steigern sollen, sind bereits im Stand der Technik beschrieben. Allerdings konnte der Stand der Technik nicht den Weg zur vorliegenden Erfindung weisen.

Bei verstärktem Kontakt polymerer Verdickungsmittel mit Gasen kann es bekanntermaßen zu u-nangenenhmen Geruchsentwicklungen kommen. Bei begasten Kosmetika ist dies besonders mißlich, da diese Produkte ja allenfalls geruchsfrei, in den meisten Fällen aber durch beigemischtes Parfum mit Wohlgeruch ausgestattet sein sollen.

Zubereitungen mit einem Gehalt an molekularem Sauerstoff, ferner enthaltend Panthenol, lösen erfindungsgemäß diese Aufgaben.

Vorteilhaft enthalten die erfindungsgemäßen Zubereitungen 5 - 25 Volumen-% Sauerstoff, vorzugsweise 10 - 20 Volumen-%, insbesondere bevorzugt 12 - 17 Volumen-.%

Vorteilhaft enthalten die erfindungsgemäßen Zubereitungen 0,1 - 5 Gew-% Panthenol, vorzugsweise 0,5 - 2,5 Gew-% Panthenol, insbesondere bevorzugt 1- 2Gew-% Panthenol.

Die erfindungsgemäße kosmetische oder dermatologische Zubereitung auf Basis einer Emulsion, insbesondere einer O/W-Emulsion, umfassend molekularen Sauerstoff ist dadurch charakterisiert, dass sie erhältlich ist durch übliches Mischen und Herstellen einer Emulsion und anschließendem Begasen der Zubereitung mit gasförmigen Sauerstoff und/oder sauerstoffhaltigen Gasen. Dabei wird die Zubereitung bei Raumtemperatur (ca. 20 bis 25 °C) in den Mischer eingespeist und anschließend begast.

Die kosmetischen und dermatologischen Zubereitungen gemäß der Erfindung können kosmetische Hilfsstoffe enthalten, wie sie üblicherweise in solchen Zubereitungen verwendet werden, z. B. Emulgatoren, Pigmente, Konservierungsmittel, Konservierungshelfer, Komplexbildner, Bakterizide, Parfüme, Substanzen zum Steigern des Schäumens, Farbstoffe, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft ist der Einsatz von Emulgatoren ausgewählt aus der Gruppe Glyceryl Stearat Citrat, Stearinsäure, Ceteareth-20, PEG-40 Stearat, Glyceryl Isostearat, Polyglyceryl-3 Diisostearate, I-sosteareth-20, Glyceryl Stearat SE, Sorbitan Stearat, Sodium Stearoyl Glutamat, Sucrose Polystearat, Potassium Cetyl Phosphat.

Der Einsatz dieser Emulgatoren erfolgt erfindungsgemäß vorteilhaft in 1 bis 5%iger Konzentration.

Es ist besonders vorteilhaft wenn als Emulgator Glyceryl Stearat Citrat in einer Einsatzkonzentration von 1,5 bis 3% in der erfindungsgemäßen Zubereitung eingesetzt wird.

Die Verwendung der erfindungsgemäßen Zubereitungen ist bevorzugt auf die Behandlung kranker, irritierter oder geschädigter Haut. Die Behandlung ist einerseits als therapeutische Behandlung möglich, wodurch die erfindungsgemäßen Zubereitungen zur Herstellung eines Arzneimittels dienen.

Insbesondere ist aber die kosmetische Behandlung der kranken, irritierten oder geschädigten Haut mit der erfindungsgemäßen Zubereitung bevorzugt.

Vorteilhaft zeigt sich die erfindungsgemäße Zubereitung in der Kinder- und Babyhautpflege. Unter den Windeln bildet sich häufig eine Rötung oder Reizung der Babyhaut (Windeldermatitis). Die Ursache ist häufig ein warm, feuchtes Milieu mit ungenügender Luftzufuhr.

Dieses Problem wird erfindungsgemäß gelöst durch eine erfindungsgemäße Babycreme, die Sauerstoff abgibt wo sonst keine hinkommt.

Durch die Verwendung von Sauerstoff bzw. kosmetischer oder topischer dermatologischer Zubereitungen mit einem wirksamen Gehalt an Sauerstoff im Sinne der vorliegenden Erfindung wird die Haut überraschend besser gegen Alterungsprozesse, Struktur- und/oder oxidative Schäden geschützt als dies mit Zubereitungen des Standes der Technik möglich wäre. Ferner wird durch die erfindungsgemäße Verwendung die Wiederherstellung des gesunden Hautzustands im Fall einer bereits bestehenden Störung und Schädigung der normalen Hauteigenschaften begünstigt.

Unter "gestörtem Erscheinungsbild der Haut" sind im Sinne der vorliegenden Erfindung insbesondere die folgenden Phänomene zu verstehen:
■ Falten und/oder Fältchen,
■ Haut- und/oder Gewebeerschlaffung,
■ Störungen der Regeneration der Haut und der Hautanhangsgebilde,
■ Durchblutungsstörungen der Haut,
■ Altersflecken, Pigmentstörungen und sogenannter "uneven skin tone"
■ sensible Haut,
■ Juckreiz,
■ Stressempfindlichkeit der Haut,
■ entzündliche und empfindliche Hautzustände,
■ trockene Hautzustände,
■ atopisches Ekzem,
■ Psoriasis
■ Unregelmäßige bzw. zunehmende Porengröße

Ferner werden bezogen auf die Hautanhangsgebilde (Haare, Nägel) insbesondere die folgenden (Alterungs-) Erscheinungen ("gestörte Erscheinungsbilder der Haare") durch die erfindungsgemä-βe Verwendung positiv beeinflußt:
- Wachstumsverlangsamung,
- Deformation,
- Spliss,
- Rissigkeit,
- vorzeitiger Verlust,
- Struktur und Farbveränderung.

Die kosmetischen oder dermatologischen Mittel gemäß der Erfindung können vorteilhaft in Form von schäumbaren Zubereitungen vorliegen, welche beispielsweise aus Aerosolbehältern entnommen und dabei aufgeschäumt werden. Erfindungsgemäß vorteilhafte Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Derartige Behälter können mit Ventilen sehr unterschiedlicher Bauart ausgestattet sein, die die Entnahme des Inhalts als Schaum ermöglichen.

Die kosmetischen oder dermatologischen Formulierungen im Sinne der vorliegenden Erfindung können wie üblich zusammengesetzt sein und dem kosmetischen oder dermatologischen Lichtschutz, ferner zur Behandlung, Pflege und Reinigung der Haut, der Lippen, sowie von Hautanhangsgebilden (Nägel und/oder Haare) und als Schminkprodukt in der dekorativen Kosmetik dienen.

Ebenso wie Emulsionen von flüssiger und fester Konsistenz als kosmetische Reinigungslotionen bzw. Reinigungscremes Verwendung finden, können auch die Zubereitungen im Sinne der vorliegenden Erfindung "Reinigungsschäume" darstellen, welche beispielsweise zum Entfernen von Schminken und/oder Make-up oder als milder Wasch- und Duschschaum - ggf. auch für unreine Haut - verwendet werden können. Derartige Reinigungsschäume können vorteilhaft ferner als sogenannte "rinse off' Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### O/W-Emulsionen

### Beispiel 1

| | **Gew.-%** |
|---|---|
| Octyldodecanol | 2 |
| Avocadoöl | 1 |
| C₁₂₋₁₅ Alkylbenzoat | 3 |
| Dimethicon | 4 |
| Cyclomethicon | 1 |
| Myristyl Myristat | 1 |
| Cetylalkohol | 1 |
| Stearinsäure | 3 |
| BHT | 0,01 |
| Ethylhexylglycerin | 2 |
| 1,2-Hexandiol | 0,2 |
| Glycerin | 5 |
| Parfum / Farbstoffe | q.s. |
| Xanthan Gum | 0,2 |
| Panthenol | 2 |
| Kreatin | 0,2 |
| Vitamin E Acetat | 0,3 |
| Tapioca Stärke | 1 |
| Phenoxyethanol | 0,5 |
| Propylparaben | 0,2 |
| Methylparaben | 0,2 |
| Natriumhydroxid | 0,15 |
| Wasser | ad 100 |
| Volumen % der Emulsion | 81 |
| Volumen % Sauerstoff | 19 |

### Beispiel 2

| | **Gew.-%** |
|---|---|
| C₁₂₋₁₅ Alkylbenzoat | 3 |
| Dimethicon | 2 |
| Butylenglycol Dicaprylat/Dicaprat | 3 |
| Squalan | 0,5 |
| Macadamiaöl | 0,75 |
| Dicaprylylether | 2 |
| Cyclomethicon | 3 |
| Cetylstearylalkohol | 3 |
| Glycerylstearatcitrat | 1,5 |
| Butyl Methoxydibenzoylmethan | 2,5 |
| Ethylhexyl Methoxycinnamat | 5 |
| EDTA | 1 |
| Methylpropandiol | 2,5 |
| 1,2-Pentandiol | 0,2 |
| Glycerin | 10 |
| Parfum / Farbstoffe | q.s. |
| Acrylates/C₁₀₋₃₀ Alkyacrylate Crosspolymer | 0,45 |
| Panthenol | 1 |
| Coenzym Q10 | 0,1 |
| Vitamin E Acetat | 0,4 |
| Phenoxyethanol | 0,2 |
| Ethylparaben | 0,2 |
| Propylparaben | 0,2 |
| Methylparaben | 0,2 |
| Natriumhydroxid | 0,2. |
| Wasser | ad 100 |
| Volumen % der Emulsion | 85 |
| Volumen % Sauerstoff | 15 |

### Beispiel 3

| | **Gew.-%** |
|---|---|
| Dicaprylylether | 3 |
| Cyclomethicon | 6 |
| Hydrogenierte Cocoglyceride | 2 |
| Cetyldimeticon | 3 |
| Caprylsäure/Caprinsäure Triglyceride | 2 |
| Cetylalkohol | 1,5 |
| Stearylalkohol | 1,5 |
| Glycerylstearatcitrat | 2 |
| EDTA | 1 |
| BHT | 0,05 |
| 1,2-Hexandiol | 0,1 |
| 1,2-Octandiol | 0,1 |
| Glycerin | 7 |
| Parfum / Farbstoffe | q.s. |
| Carbomer | 0,6 |
| Panthenol | 1,3 |
| Vitamin E Acetat | 0,5 |
| Nylon-12 | 2 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |
| Volumen % der Emulsion | 89 |
| Volumen % Sauerstoff | 11 |

### Beispiel 4

| | **Gew.-%** |
|---|---|
| Nachtkerzenöl | 1 |
| Isohexadecan | 0,5 |
| Dimethicon/Vinyl Dimethicon Crosspolymer | 0,75 |
| Cylclopentasiloxan | 0,25 |
| Cyclomethicon | 6 |
| Shea Butter | 2 |
| Vaseline | 1 |
| Jojobaöl | 0,5 |
| Caprylsäure/Caprinsäure Triglyceride | 3 |
| Cetylalkohol | 1,5 |
| Myristylalkohol | 1 |
| Stearinsäure | 2,5 |
| Ethylhexylglycerin | 2 |
| 1,2-Hexandiol | 0,1 |
| 1,2-Pentandiol | 0,2 |
| Glycerin | 6,5 |
| Parfum / Farbstoffe | q.s. |
| Acrylates/C₁₀₋₃₀ Alkyl Acrylate Crosspolymer | 0,4 |
| Panthenol | 5 |
| Vitamin E Acetat | 0,4 |
| DHA | 2 |
| Phenoxyethanol | 0,3 |
| Ethylparaben | 0,15 |
| Propylparaben | 0,15 |
| Methylparaben | 0,15 |
| Natriumhydroxid | q.s. |
| Wasser | ad 100 |
| Volumen % der Emulsion | 87 |
| Volumen % Sauerstoff | 13 |

### Beispiel 5

| | **Gew.%** |
|---|---|
| Dicaprylylcarbonat | 1,5 |
| C₁₂₋₁₅ Alkylbenzoat | 1,5 |
| Dimethicon | 2 |
| Cyclomethicon | 4 |
| Cetylalkohol | 1,5 |
| Cetylstearylalkohol | 1,5 |
| Polyglyceryl-3 Methylglucose Distearat | 3 |
| Butyl Methoxydibenzoylmethan | 1 |
| Ethylhexyl Methoxycinnamat | 4 |
| Titandioxid | 1 |
| Methylpropandiol | 0,75 |
| 1,2-Octandiol | 0,15 |
| Glycerin | 7,5 |
| Parfum / Farbstoffe | q.s. |
| Xanthangummi | 0,5 |
| Panthenol | 0,6 |
| BHT | 0,1 |
| Vitamin E Acetat | 1 |
| DHA | 1 |
| Silica + Cl 77891 + Cl 77491 | 2 |
| Phenoxyethanol | 0,5 |
| Ethylparaben | 0,1 |
| Propylparaben | 0,1 |
| Methylparaben | |
| Natriumhydroxid | q.s. |
| Wasser | ad 100 |
| Volumen % der Emulsion | 80 |
| Volumen % Sauerstoff | 20 |

### Beispiel 6

| | **Gew.-%** |
|---|---|
| Hydrogenierte Cocoglyceride | 3 |
| Cetyldimeticon | 2 |
| Caprylsäure/Caprinsäure Triglyceride | 4 |
| Stearylalkohol | 1 |
| Glycerylstearat SE | 3 |
| Ethylhexyl Methoxycinnamat | 3 |
| Ethylhexylglycerin | 1 |
| Methylpropandiol | 1 |
| 1,2-Hexandiol | 1 |
| 1,2-Pentandiol | 1 |
| 1,2-Octandiol | 1 |
| Glycerin | 12,5 |
| Parfum / Farbstoffe | q.s. |
| Carbomer | 0,3 |
| Panthenol | 4,4 |
| Vitamin E Acetat | 1,2 |
| Folsäure | 0,01 |
| Phenoxyethanol | 0,2 |
| Ethylparaben | 0,15 |
| Propylparaben | 0,15 |
| Methylparaben | 0,15 |
| Natriumhydroxid | q.s. |
| Wasser | ad 100 |
| Volumen % der Emulsion | 75 |
| Volumen % Sauerstoff | 25 |

Die Zubereitung der beispielsgemäßen Zubereitungen erfolgt gemäß nachstehendem Schema:

Gegebenenfalls Vordispergierung des anorganischen Gelbildners und Quellung des Hydrokolloides unter Rühren in der Wasserphase.

Vereinigung der auf 75 °C aufgeheizten Fettphase mit der auf 70 °C aufgeheizten Wasserphase.

Gegebenenfalls Zugabe der partikulären hydrophoben, hydrophobisierten Festkörpersubstanzen unter Rühren.

Homogenisierung mit einer Zahnkranzdispergiermaschine (Rotor-Stator-Prinzip) bei 65 °C. 45 min Rühren unter Vakuum und Kühlung. Zugabe der Zusatzstoffe bei 30 °C (Parfüm, Wirkstoffe).

Homogenisierung mit einer Zahnkranzdispergiermaschine (Rotor-Stator-Prinzip) bei 27 °C.

Begasung der Emulsion bei 7-8 bar mittels eines dynamischen Schaumgenerators (z.B. von der Firma HANSA INDUSTRIE MIXER) mit Sauerstoff.

## Patentansprüche

1. Zubereitungen mit einem Gehalt an molekularem Sauerstoff, ferner enthaltend Panthenol.

2. Zubereitungen nach Anspruch 1, in Form von O/W-oder W/O-Emulsionen vorliegend.

3. Zubereitungen nach Anspruch 1, enthaltend 5- 25 Volumen-% Sauerstoff, vorzugsweise 10 - 20 Volumen-%, insbesondere bevorzugt 12 - 17 Volumen-.%

4. Zubereitungen nach Anspruch 1, enthaltend 0,1 - 5 Gew-% Panthenol, vorzugsweise 0,5 - 2,5 Gew-% Panthenol, insbesondere bevorzugt 1-2Gew-% Panthenol.

5. Verwendung von Panthenol zur Verminderung oder Maskierung unerwünschter Gerüche mit Sauerstoff begaster Emulsionen
